# EUROPÄISCHE PATENTSCHRIFT

(11) **EP 2 421 637 B1**
(45) Veröffentlichungstag und Bekanntmachung des Hinweises auf die Patenterteilung: **08.05.2013**
(21) Anmeldenummer: 10710345.9
(22) Anmeldetag: 26.03.2010
(51) Int. Cl.: B01J 4/00

(54) **DOSIERRING**
METERING RING
BAGUE DOSEUSE

(30) Priorität: 23.04.2009 DE 102009002592
(43) Veröffentlichungstag der Anmeldung: 29.02.2012
(73) Patentinhaber: Evonik Röhm GmbH, 64293 Darmstadt (DE)
(72) Erfinder: SARTORELLI, Lorenza, 64372 Ober-Ramstadt (DE); PERL, Andreas, 67240 Bobenheim-Roxheim (DE); GROPP, Udo, 83093 Bad Endorf (DE); SELBACH, Arndt, 50374 Erftstadt (DE); SOHNEMANN, Stefanie, 51491 Overath (DE); GRÖMPING, Matthias, 64319 Pfungstadt (DE); MNICH, Norbert, 63526 Erlensee (DE); MERTZ, Thomas, 64625 Bensheim (DE)
(86) Internationale Anmeldenummer: PCT/EP2010/053962
(87) Internationale Veröffentlichungsnummer: WO 2010/121882

(56) Entgegenhaltungen:
- DE-A1- 10 144 681
- DE-A1-102004 055 425
- JP-A- 63 023 734
- US-A- 2 548 759
- US-A1- 2003 152 500

## Beschreibung

(Meth)acrylsäure und (Meth)acrylsäureester stellen wichtige Erzeugnisse der chemischen Industrie dar, welche als Ausgangsstoffe für viele wichtige Produkte dienen. Daher ist eine maximale Ausbeute, eine besonders hohe Reinheit bei geringen Herstellungskosten wesentlich für den wirtschaftlichen Erfolg eines Herstellungsprozesses für ein derart wichtiges Produkt. Schon relativ kleine Verbesserungen hinsichtlich der Ausbeuten, der Standzeiten der Anlagen oder ähnlicher Verfahrensmerkmale führen zu einem bedeutenden Fortschritt hinsichtlich der Menge an unerwünschten Nebenprodukten und der Herstellkosten.

Das zur Herstellung von Methacrylsäure eingesetzte Methacrylamid kann vorzugsweise durch das so genannte ACH-Verfahren erhalten werden. Ausgehend von Blausäure und Aceton wird in einem ersten Schritt Acetoncyanhydrin hergestellt, welches anschließend zum Methacrylamid umgesetzt wird. Diese Schritte sind unter anderem in US 7,253,307, EP-A-1 666 451 und PCT/EP2007 059092 dargestellt.

Acetoncyanhydrin wird nach allgemein bekannten Verfahren (siehe beispielsweise Ullmanns Enzyklopädie der technischen Chemie, 4. Auflage, Band 7) hergestellt. Häufig werden dabei als Reaktionspartner Aceton und Blausäure eingesetzt. Bei der Umsetzung handelt es sich um eine exotherme Reaktion. Um einer Zersetzung des im Rahmen dieser Reaktion gebildeten Acetoncyanhydrins entgegenzuwirken, wird üblicherweise die Reaktionswärme durch eine geeignete Vorrichtung abgeführt. Die Umsetzung kann dabei grundsätzlich als Batchprozess oder als kontinuierliches Verfahren geführt werden, sofern eine kontinuierliche Fahrweise bevorzugt ist, wird die Umsetzung häufig in einem Schlaufenreaktor, der entsprechend eingerichtet ist, durchgeführt.

Das über verschiedene bekannte Herstellverfahren dargestellte Acetoncyanhydrin wird üblicherweise einer destillativen Aufbereitung unterzogen. Dabei wird das stabilisierte rohe Acetoncyanhydrin über eine entsprechende Kolonne von niedrigsiedenden Bestandteilen befreit. Ein geeignetes Destillationsverfahren kann beispielsweise über nur eine Kolonne geführt werden. Es ist jedoch ebenfalls möglich, im Rahmen einer entsprechenden Aufreinigung von rohem Acetoncyanhydrin eine Kombination von zwei oder mehr Destillationskolonnen auch kombiniert mit einem Fallfilmverdampfer einzusetzen. Weiterhin können zwei oder mehrere Fallfilmverdampfer oder auch zwei oder mehrere Destillationskolonnen miteinander kombiniert werden.

Das rohe Acetoncyanhydrin kommt in der Regel mit einer Temperatur von etwa 0 bis etwa 15°C, beispielsweise einer Temperatur von etwa 5 bis etwa 10°C aus der Lagerung zur Destillation. Grundsätzlich kann das rohe Acetoncyanhydrin direkt in die Kolonne eingeführt werden. Es hat sich jedoch in einigen Fällen bewährt, wenn zunächst das rohe, kühle Acetoncyanhydrin über einen Wärmetauscher einen Teil der Wärme des bereits destillativ gereinigten Produktes übernimmt. Daher wird im Rahmen einer weiteren Ausführungsform des hier beschriebenen Verfahrens das rohe Acetoncyanhydrin über einen Wärmetauscher auf eine Temperatur von etwa 60 bis 80°C erhitzt.

Die destillative Reinigung des Acetoncyanhydrin erfolgt über eine Destillationskolonne mit mehr als 5, bevorzugt mehr als 10 Böden oder über eine Kaskade von zwei oder mehr entsprechend geeigneter Destillationskolonnen. Die Beheizung des Kolonnensumpfes erfolgt vorzugsweise mit Dampf. Es hat sich als vorteilhaft herausgestellt, wenn die Sumpftemperatur eine Temperatur von 140°C nicht übersteigt, gute Ausbeuten und eine gute Reinigung haben sich erzielen lassen, wenn die Sumpftemperatur nicht größer als etwa 130°C oder nicht höher als etwa 110°C ist. Die Temperaturangaben beziehen sich dabei auf die Wandtemperatur des Kolonnensumpfes.

Das rohe Acetoncyanhydrin wird im oberen Drittel der Kolonne dem Kolonnenkörper zugeführt. Die Destillation wird vorzugsweise bei verringertem Druck, beispielsweise bei einem Druck von etwa 50 bis etwa 900 mbar, insbesondere etwa 50 bis etwa 250 mbar und mit guten Ergebnissen zwischen 50 bis etwa 150 mbar durchgeführt.

Am Kopf der Kolonne werden gasförmige Verunreinigungen, insbesondere Aceton und Blausäure, entnommen, die abgetrennten gasförmigen Stoffe werden über einen Wärmetauscher oder eine Kaskade von zwei oder mehr Wärmetauschern gekühlt. Hierbei wird vorzugsweise eine Solekühlung mit einer Temperatur von etwa 0 bis etwa 10°C eingesetzt. Dabei wird den gasförmigen Inhaltsstoffen der Brüden die Gelegenheit gegeben, zu kondensieren. Die erste Kondensationsstufe kann beispielsweise bei Normaldruck stattfinden. Es ist jedoch ebenso möglich und hat sich in einigen Fällen als vorteilhaft erwiesen, wenn diese erste Kondensationsstufe unter verringertem Druck, vorzugsweise bei dem Druck, der im Rahmen der Destillation vorherrscht, erfolgt. Das Kondensat wird in einen gekühlten Auffangbehälter weitergeleitet und dort bei einer Temperatur von etwa 0 bis etwa 15°C, insbesondere bei etwa 5 bis etwa 10°C gesammelt.

Die im Rahmen des ersten Kondensationsschrittes nicht kondensierenden gasförmigen Verbindungen werden über eine Vakuumpumpe aus dem Unterdruckraum entfernt. Hierbei ist grundsätzlich eine beliebige Vakuumpumpe einsetzbar. Es hat sich jedoch in vielen Fällen als vorteilhaft erwiesen, wenn eine Vakuumpumpe eingesetzt wird, die aufgrund ihrer Bauweise nicht zum Eintrag flüssiger Verunreinigungen in den Gasstrom führt. Vorzugsweise werden hier daher beispielsweise trocken laufende Vakuumpumpen eingesetzt.

Der auf der Druckseite der Pumpe entweichende Gasstrom wird über einen weiteren Wärmetauscher geführt, der vorzugsweise mit Sole bei einer Temperatur von etwa 0 bis etwa 15°C gekühlt wird. Hierbei kondensierende Inhaltsstoffe werden ebenfalls in dem Sammelbehälter gesammelt, der bereits die unter Vakuumbedingungen gewonnenen Kondensate auffängt. Die auf der Druckseite der Vakuumpumpe durchgeführte Kondensation kann beispielsweise durch einen Wärmetauscher, jedoch auch mit einer Kaskade von zwei oder mehr seriell parallel angeordneten Wärmetauschern erfolgen. Nach diesem Kondensationsschritt verbleibende gasförmigen Stoffen werden abgeführt und einer beliebigen weiteren Verwertung, beispielsweise einer thermischen Verwertung, zugeführt.

Die gesammelten Kondensate können ebenfalls beliebig weiterverwertet werden. Es hat sich jedoch als unter ökonomischen Gesichtspunkten äußerst Vorteilhaft erwiesen, die Kondensate in die Reaktion zur Herstellung von Acetoncyanhydrin zurückzuführen. Dies erfolgt vorzugsweise an einer oder mehreren Stellen, die Zugang zum Schlaufenreaktor ermöglichen. Die Kondensate können grundsätzlich eine beliebige Zusammensetzung aufweisen, sofern sie die Herstellung des Acetoncyanhydrins nicht stören. In vielen Fällen wird die überwiegende Menge des Kondensates jedoch aus Aceton und Blausäure bestehen, beispielsweise in einem molaren Verhältnis von etwa 2:1 bis etwa 1:2, häufig in einem Verhältnis von etwa 1:1.

Das aus dem Sumpf der Destillationskolonne gewonnene Acetoncyanhydrin wird zunächst über einen ersten Wärmetauscher durch das zugeführte, kalte rohe Acetoncyanhydrin auf eine Temperatur von etwa 40 bis etwa 80°C abgekühlt. Anschließend wird das Acetoncyanhydrin über einen mindestens einen weiteren Wärmetauscher auf eine Temperatur von etwa 30 bis etwa 35°C gekühlt und ggf. zwischengelagert.

Im Rahmen eines weiteren Verfahrenselements wird Acetoncyanhydrin einer Hydrolyse unterzogen. Dabei bildet sich bei verschiedenen Temperaturstufen nach einer Reihe von Reaktionen als Produkt Methacrylamid.

Die Umsetzung wird in einer dem Fachmann bekannten Weise durch eine Reaktion zwischen konzentrierter Schwefelsäure und Acetoncyanhydrin bewirkt. Die Umsetzung ist exotherm, so dass in vorteilhafter Weise Reaktionswärme aus dem System abgeführt wird.

Die Umsetzung kann auch hier wieder im Batchverfahren oder in kontinuierlichen Verfahren durchgeführt werden. Letzteres hat sich in vielen Fällen als vorteilhaft erwiesen. Sofern die Umsetzung im Rahmen eines kontinuierlichen Verfahrens durchgeführt wird, hat sich der Einsatz von Schlaufenreaktoren bewährt. Schlaufenreaktoren sind in der Fachwelt bekannt. Diese können insbesondere in Form von Rohrreaktoren mit Rückführung ausgestaltet sein. Die Umsetzung kann beispielsweise in nur einem Schlaufenreaktor erfolgen. Es kann jedoch vorteilhaft sein, wenn die Umsetzung in einer Kaskade von zwei oder mehreren Schlaufenreaktoren durchgeführt wird.

Ein geeigneter Schlaufenreaktor weist im Rahmen des beschriebenen Verfahrens eine oder mehrere Zufuhrstellen für Acetoncyanhydrin, eine oder mehrere Zufuhrstellen für konzentrierte Schwefelsäure, einen oder mehrere Gasabscheider, einen oder mehrere Wärmetauscher und einen oder mehrere Mischer auf. Der Schlaufenreaktor kann weitere Bestandteile, wie Fördermittel, Pumpen, Kontrollelemente usw. umfassen.

Die Hydrolyse von Acetoncyanhydrin mit Schwefelsäure ist, wie bereits beschrieben, exotherm. Parallel zur Hauptreaktion finden mehrere Nebenreaktionen statt, die zur Senkung der Ausbeute führen. In dem bevorzugten Temperaturbereich spielt die Zersetzung von Acetoncyanhydrin, ebenfalls eine exotherme und schnelle Reaktion, eine wesentliche Rolle. Die im Rahmen der Reaktion anfallende Reaktionswärme muss dem System jedoch zumindest weitgehend entzogen werden, da mit zunehmender Betriebstemperatur und steigender Verweilzeit die Ausbeute sinkt. Zwar ist es grundsätzlich möglich mit entsprechenden Wärmetauschern eine schnelle und umfassende Abfuhr der Reaktionswärme zu erzielen. Es kann jedoch auch nachteilig sein, das Gemisch vor der Dosierung von Acetoncyanhydrin zu sehr zu kühlen, da sowohl für eine Vermischung als auch für eine effiziente Wärmeabfuhr eine hohe Turbulenz notwendig ist. Da mit sinkender Temperatur die Viskosität des Anrührgemisches stark ansteigt,sinkt entsprechend die Strömungsturbulenz, teilweise bis in den Laminarbereich, was im Wärmentauscher zu einer ineffizienteren Wärmeabfuhr und bei der Azetoncyanhydrin Dosierung zu einer langsameren und inhomogeneren Mischung führt.

Gefordert ist eine schnelle Vermischung von Acetoncyanhydrin und Reaktionsgemisch, da das Acetoncyanhydrin reagieren soll bevor es aufgrund der Aufwärmung zersetzt wird. Eine feine Tropfenverteilung des Reaktants, was eine große spezifische Grenzoberfläche bedeutet, bevorzugt die gewünschte Reaktion auf der Tropfenoberfläche gegenüber der Aufwärmung des Tropfenvolumens mit darauf folgender Zersetzung. Eine feine Verteilung von Acetoncyanhydrin hat sich als vorteilhaft gezeigt, da die Reaktion an der Tropfenoberfläche stattfindet.

Darüber hinaus können zu geringe Temperaturen im Reaktionsgemisch zu einer Kristallisation von Inhaltsstoffen des Reaktionsgemischs an den Wärmetauschern führen. Dadurch wird der Wärmeübergang weiter verschlechtert, wodurch sich eindeutiger Ausbeuterückgang verzeichnen lässt. Darüber hinaus kann der Schlaufenreaktor nicht mit den optimalen Mengen an Reaktanden beschickt werden, so dass insgesamt die Effizienz des Verfahrens leidet.

Aufgabe der Erfindung ist es deshalb die Zuführung und die feine Verteilung von fließfähigen Medien oder Gasen in Rohrleitungen oder Rohrreaktoren zu verbessern, idealerweise auch Mischvorgänge zu verbessern.

Diese Aufgabe wird gelöst durch eine Vorrichtung zur Dosierung von fließfähigen Medien oder Gasen, dadurch gekennzeichnet, dass ein oder mehrere Dosierringe mit Dosierstellen [11] versehen in Rohrleitungen, Rohrreaktoren oder Schlaufenreaktoren eingesetzt werden, wobei der oder die Dosirringe außenliegend sind, eine umlaufende Verteilerhammer aufweisen und die innere wend der Dosterringe von 2 bis 20 lujektionskanälen durch ist.

Überraschend wurde gefunden, dass die mit dem Dosierring ermöglichte Zufuhr und feine Verteilung eines fließfähigen Mediums oder Gases über den gesamten Rohrumfang bzw. die Rohrquerschnittsfläche zu einer wesentlichen Verbesserung des Mischvorganges kommt. Auf kurzem Weg können große Mengen vermischt werden.

Der erfindungsgemäße Dosierring kann verschiedene Ausführungsformen haben. Bespielsweise können viele kleine Dosierstellen [11] in den Ring eingelassen sein, oder wenige große Dosierstellen (Zeichnung 1). Die Dosierstellen können auch über Röhrchen [13] in das Innere des Rohres ragen (Zeichnung 2), in besonderen Ausführungsformen auch verschieden lange Röhrchen.

Der Dosierring kann je nach Dosieraufgabe gekühlt oder erwärmt werden.

Hier liegt ein weiterer Vorteil des erfindungsgemäßen, außenliegenden Ringes, der nicht durch das umgebende Medium erhitzt wird wie z.B. eine Dosierlanze, die in dem Rohr steckt. Weiterhin könnte man durch diesen Ring das Acetoncyanhydrin kühlen, was z.B. in einer Dosierlanze konstruktiv sehr viel aufwendiger wäre.

Eine besondere Ausführungsform ist ein Dosierring, bei dem die Zudosierung unter Überdruck erfolgt.

Die Vorrichtung kann jede geeignete Raumform annehmen, bevorzugt ist sie in Ringform konstruiert. Hierbei können auch Doppel- oder Mehrfachringe eingesetzt werden.

Der Dosierring eignet sich besonders für den Einsatz in kontinuierlichen Prozessen. Bevorzugt wird der Dosierring bei der kontinuierlichen Herstellung von Methacrylamid durch Hydrolyse von Acetoncyanhydrin mit Schwefelsäure eingesetzt. Ein anderes Einsatzgebiet wäre beispielsweise die Herstellung von Acetoncyanhydrin aus Aceton und Blausäure.

Erfindungsgemäß erfolgt die Umsetzung kontinuierlich in einem Rohrreaktor oder Schlaufenreaktor. Die Begriffe "kontinuierlich" und "Rohrreaktor" sind in der Fachwelt bekannt. Unter einer kontinuierlichen Reaktion sind insbesondere Umsetzungen zu verstehen, bei denen über einen längeren Zeitraum Edukte zugefügt und Produkte aus dem Reaktionsgemisch entfernt werden. Rohrreaktoren umfassen mindestens einen röhrenförmigen Bereich, in dem die Reaktion erfolgen kann. Diese Reaktoren weisen üblich einen relativ einfachen Aufbau auf, so dass die Investitionskosten vergleichsweise gering sind.

Die Edukte können über eine Pumpe in den Rohrreaktor eingeleitet werden. Zur Vermeidung von wartungsbedingten Betriebsunterbrechungen können auch zwei oder mehr Pumpen vorgesehen sein, die parallel geschaltet werden können. Das Mischen der Edukte mit Dosierring kann zweckmäßig in Strömungsrichtung gesehen vor den Pumpen, also auf der Pumpensaugseite, erfolgen, wobei die Anlage besonders bevorzugt in dem Bereich zwischen den Pumpen und dem Rohrreaktor keine weiteren Einbauten zum Mischen aufweist. Der Dosierring kann aber auch Bestandteil der Pumpe und in das Pumpengehäuse integriert sein. Durch diese Maßnahmen können überraschende Vorteile hinsichtlich der Betriebssicherheit und der Standzeiten der Anlage sowie in Bezug auf die Ausbeute und die Reinheit des Produkts erzielt werden.

Die Komponenten der Anlage, die mit korrosiven Stoffen in Berührung kommen, insbesondere der Rohrreaktor, die Pumpen und die Phasentrenner sind aus geeigneten Materialien, beispielsweise einem säureresistenten Metall, wie beispielsweise Zirkonium, Tantal, Titan oder rostfreien Stahl oder einem beschichteten Metall, das beispielsweise eine Emailschicht oder eine Zirkonschicht aufweist, aufgebaut. Weiterhin können auch Kunststoffe, beispielsweise PTFE ummantelte Bauteile, graphitisierte Komponenten oder Werkstücke aus Graphit, insbesondere in Pumpen eingesetzt werden.

Im Rahmen einer Ausgestaltung des Verfahrens wird aus einem Strom von Acetoncyanhydrin ein Teil, vorzugsweise etwa zwei Drittel bis etwa drei Viertel, des Volumenstroms in einen ersten Schlaufenreaktor eingeführt. Vorzugsweise weist ein erster Schlaufenreaktor einen oder mehrere Wärmetauscher, eine oder mehrere Pumpen, einen oder mehrere Mischelemente und einen oder mehrere Gasabscheider auf. Die den ersten Schlaufenreaktor durchlaufenden Umwälzströme liegen vorzugsweise im Bereich von etwa 50 bis 650 m³/h, bevorzugt in einem Bereich von 100 bis 500 m³/h und darüber hinaus bevorzugt in einem Bereich von etwa 150 bis 450 m³/h. In einem auf den ersten Schlaufenreaktor folgenden mindestens einem weiteren Schlaufenreaktor liegen die Umwälzströme vorzugsweise in einem Bereich von etwa 40 bis 650 m³/h, bevorzugt in einem Bereich von 50 bis 500 m³/h und darüber hinaus bevorzugt in einem Bereich von etwa 60 bis 350 m³/h. Weiterhin sind als Temperaturdifferenz über den Wärmetauscher etwa 1 bis 20° C bevorzugt, wobei etwa 2 bis 7°C besonders bevorzugt sind.

Die Zufuhr des Acetoncyanhydrin, erfindungsgemäß über einen Dosierring, kann grundsätzlich an beliebiger Stelle in den Schlaufenreaktor erfolgen. Es hat sich jedoch als vorteilhaft erwiesen, wenn die Zufuhr in ein Mischelement, beispielsweise in einen Mischer mit beweglichen Teilen oder einen Statikmischer oder an gut durchmischter Stelle erfolgt. Die Zufuhr der Schwefelsäure erfolgt vorteilhafterweise vor der Acetoncyanhydrin Zugabe. Ansonsten ist es jedoch ebenfalls möglich, die Schwefelsäure an beliebiger Stelle in den Schlaufenreaktor einzuführen.

Der erfindungsgemäße Dosierring wird zur Einspeisung des Mediums, beispielsweise der Schwefelsäure oder des Acetoncyanhydrins kurz vor oder in einer Pumpe eingesetzt. Somit wird die hochturbulente Strömung im Pumpengehäuse zur Mischung der Edukte und dadurch eine Fördermaschine gleichzeitig als Mischmaschine genutzt. Somit wird die Mischleistung der Pumpe vorteilhaft ausgenutzt.

Das Verhältnis der Reaktanden im Schlaufenreaktor wird so gesteuert, dass ein Überschuss Schwefelsäure vorliegt. Der Überschuss an Schwefelsäure beträgt, bezüglich des molaren Verhältnisses der Inhaltsstoffe, im ersten Schlaufenreaktor etwa 1,8:1 bis etwa 3:1 und im letzten Schlaufenreaktor etwa 1,1:1 bis etwa 2:1.

In einigen Fällen hat es sich als vorteilhaft erwiesen, mit einem derartigen Überschuss an Schwefelsäure die Reaktion im Schlaufenreaktor zu betreiben. Die Schwefelsäure kann hier beispielsweise als Lösemittel dienen und die Viskosität des Reaktionsgemischs niedrig halten, wodurch eine schnellere Abfuhr von Reaktionswärme und eine niedrigere Temperatur des Reaktionsgemischs gewährleistet werden kann. Dies kann deutliche Ausbeutevorteile mit sich bringen. Die Temperatur im Reaktionsgemisch beträgt etwa 85 bis etwa 150°C.

Die Wärmeabfuhr wird durch einen oder mehrere Wärmetauscher im Schlaufenreaktor gewährleistet. Es hat sich dabei als vorteilhaft erwiesen, wenn die Wärmetauscher über eine geeignete Sensorik zum Einstellen der Kühlleistung verfügen, um eine zu starke Kühlung des Reaktionsgemischs aus den oben genannten Gründen zu verhindern. So kann es beispielsweise vorteilhaft sein, den Wärmeübergang im Wärmetauscher oder in den Wärmetauschern punktförmig oder kontinuierlich zu messen und daran die Kühlleistung der Wärmetauscher anzupassen. Dies kann beispielsweise über das Kühlmittel selbst geschehen. Es ist auch ebenso möglich durch entsprechende Variation der Zugabe der Reaktionspartner und durch die Erzeugung von mehr Reaktionswärme eine entsprechende Erhitzung des Reaktionsgemischs zu erreichen. Auch eine Kombination von beiden Möglichkeiten ist denkbar. Bevorzugt verfügt der Schlaufenreaktor darüber hinaus über mindestens einen Gasabscheider. Über den Gasabscheider kann einerseits dem Schlaufenreaktor kontinuierlich gebildetes Produkt entnommen werden. Andererseits lassen sich im Rahmen der Reaktion gebildete Gase so dem Reaktionsraum entziehen. Als Gas bildet sich hauptsächlich Kohlenmonoxid. Das aus dem Schlaufenreaktor entnommene Produkt wird vorzugsweise in einen zweiten Schlaufenreaktor überführt. In diesen zweiten Schlaufenreaktor wird das Schwefelsäure und Metharcylsäureamid beinhaltende Reaktionsgemisch wie es durch die Reaktion im ersten Schlaufenreaktor erhalten wurde, mit dem verbleibenden Teilstrom an Acetoncyanhydrin umgesetzt. Hierbei reagiert der Überschuss an Schwefelsäure aus dem ersten Schlaufenreaktor, oder zumindest ein Teil der überschüssigen Schwefelsäure, mit dem Acetoncyanhydrin unter weiterer Bildung von Sulfoxy-Isobuttersäure-Amid (SIBA). Die Durchführung der Reaktion in zwei oder mehr Schlaufenreaktoren weist den Vorteil auf, dass aufgrund des Schwefelsäureüberschusses im ersten Schlaufenreaktor die Pumpbarkeit des Reaktionsgemischs und damit der Wärmeübergang und letztendlich die Ausbeute verbessert werden. Im zweiten Schlaufenreaktor sind wiederum mindestens ein Mischelement, mindestens ein Wärmetauscher und mindestens ein Gasabscheider angeordnet. Die Reaktionstemperatur im zweiten Schlaufenreaktor beträgt ebenfalls etwa 90 bis etwa 120°C.

Das Problem der Pumpbarkeit des Reaktionsgemischs, des Wärmeübergangs und einer möglichst geringen Reaktionstemperatur stellt sich im jedem weiteren Schlaufenreaktor genauso wie im ersten. Deshalb verfügt vorteilhafter Weise auch der zweite Schlaufenreaktor über einen Wärmetauscher, dessen Kühlleistung durch entsprechende Sensorik geregelt werden kann.

Die Zufuhr des Acetoncyanhydrins erfolgt wiederum in einem geeigneten Mischelement, vorzugsweise in einen Statikmischer oder dem erfindungsgemäßen Dosierring. Aus dem Gasabscheider des zweiten Schlaufenreaktors kann das Produkt entnommen und zur Vervollständigung der Umsetzung und zur Bildung des Methacrylsäureamids auf eine Temperatur von etwa 130 bis etwa 180°C erhitzt werden.

Die Erhitzung wird vorzugsweise derart durchgeführt, dass die Maximaltemperatur nur für einen möglichst kurzen Zeitraum, beispielsweise für eine Zeit von etwa einer Minute bis etwa 30 Minuten, insbesondere für eine Zeit von etwa zwei bis etwa acht oder etwa drei bis etwa fünf Minuten erreicht wird. Dies kann grundsätzlich in beliebigen Apparaturen zur Erzielung einer derartigen Temperatur für einen derart kurzen Zeitraum erfolgen. Beispielsweise kann die Energiezufuhr auf konventionellem Wege durch elektrische Energie oder durch Dampf erfolgen. Es ist jedoch ebenso möglich, die Energie durch elektromagnetische Strahlung, beispielsweise durch Mikrowellen zuzuführen.

Es hat sich in verschiedenen Fällen als vorteilhaft erwiesen, wenn der Erhitzungsschritt in einem Wärmetauscher mit zwei oder mehrstufiger Anordnung von Rohrwendeln, die vorzugsweise in einer mindestens doppelten, gegenläufigen Anordnung vorliegen können, erfolgt. Dabei wird das Reaktionsgemisch schnell auf eine Temperatur von etwa 130 bis 180°C erhitzt.

Die so erhältliche Amidlösung weist in der Regel eine Temperatur von mehr als 100°C, üblicherweise eine Temperatur von etwa 130 bis 180°C auf. Eine Kühlung auf Temperaturen kleiner 130°C ist ebenfalls möglich.

Neben dem Einsatz der erfindungsgemäßen Vorrichtung in chemischen Verfahren bieten sich viele weitere Einsatzmöglichkeiten an.

Beispielsweise kann der Dosierring auch in Überlandleitungen für den Erdöltransport verwendet werden. In regelmäßigen Abständen müssen dem Rohöl Fließverbesserer zugefügt werden. An diesen Einspeisungsstellen wird zumeist über eine Düse der Fließverbesserer zugegeben. Der große Volumenstrom drückt das zudosierte Medium vorrangig an die Innenseite des Rohres, die Umsetzung erfolgt schlecht und dann auch nur über eine lange Wegstrecke. Mit dem erfindungsgemäßen Dosierring kann sichergestellt werden, dass der Fließverbesserer über den gesamten Rohrquerschnitt dem Rohöl beigemischt wird.

Eine ähnliche Anwendung bieten die bei der Öl- und Gasgewinnung notwendigen Chemikalienzugaben.

Ein weiterer Gegenstand der Erfindung ist die Verwendung der erfindungsgemäßen Dosiervorrichtung in chemischen Verfahren, bevorzugt in Verfahren, bei denen eine schnelle Durchmischung und eine feine Verteilung eines Mediums erforderlich sind. Idealerweise wird am Punkt der Zudosierung das zugeführte Medium komplett mit dem vorbeiströmenden Medium umgesetzt bzw. vermischt. Bei der Zudosierung beispielsweise eines Tropfens Flüssigkeit bis zur Feinvermischung mit dem anderen Medium sollte eine möglichst kurze Strecke bis zum Mischpunkt zurückgelegt werden.

Es wurde gefunden, dass der erfindungsgemäße Dosierring bei der Zufuhr von fließfähigen Medien oder Gasen an einer Einspeisungsstelle eine fast ideale Durchmischung bzw. Umsetzung ermöglicht.

Gegenüber den herkömmlichen Dosiervorrichtungen, die eine Dosierstelle haben, erfolgt bei der erfindungsgemäßen Dosiervorrichtung über einen Ring mit mehreren Dosierstellen eine gleichmäßigere Verteilung über den Rohrquerschnitt. Damit wird das Mischergebnis wesentlich verbessert und gleichzeitig die Mischzeit verkürzt. Die innere Wand des Dosierringes wird von 2 bis 20 Injektionskanälen durchsetzt. Vorzugsweise werden 16 gleichmäßig auf den Umfang verteilte Injektionskanäle genutzt. Diese können einzeln oder gemeinsam unter einem Winkel von 1° bis 179°, bevorzugt von 20° bis 120°, besonders bevorzugt von 60°, gegenüber der Innenwand der Rohleitung geneigt sein. Bei den bekannten Dosiervorrichtungen mit einer Dosierstelle kommt es bei starken Strömungen innerhalb des Rohres dazu, dass das zudosierte Medium durch das vorbeiströmende Medium an die Rohrwand gedrückt wird, und somit keine bzw. nur eine sehr schlechte Durchmischung stattfindet. Daher können diese Zudosierungsstellen nicht gleichzeitig zur Mischung verwendet werden. Nach den Dosierstellen müssen dann zusätzlich noch Mischvorgänge eingeleitet werden. Dies erfolgt durch den Einbau statischer Mischelemente oder den Einbau von Pumpen oder ähnliches.

Die bekannten Zudosierungen entlang eines Rohres mit mehreren Dosierstellen sind für viele chemische Verfahren ungeeignet, da über den langen Mischweg die chemische Umsetzung negativ beeinflusst wird. Durch den langen Mischweg kommt es zu thermischen Zersetzungen und damit wird die Ausbeute verschlechtert.

Es kann auf den Einsatz von statischen Mischelementen verzichtet werden, die bei der Verwendung von korrosiven Medien zudem auch noch regelmäßig ausgetauscht werden müssen und somit zu Stillstandzeiten führen. Der Einsatz statischer Mischelemente führt immer auch zu unerwünschten Druckverlusten.

Besonders vorteilhaft ist es, den Dosierring vor einer Pumpe zu installieren. Idealerweise wird der Dosierring direkt vor dem Saugstutzen der Umwälzpumpe positioniert. Damit können die Turbulenzen in der Pumpe zur Vermischung genutzt werden.

Ebenso kann der Dosierring in der Kreiselpumpe eingesetzt werden. Idealerweise nahe dem Punkt, an dem die kinetische Energie am größten ist, um eine ideale Durchmischung zu erwirken. Eine Zudosierung in der Mitte der Pumpe führt dazu, dass der Weg zum Pumpenausgang lang und damit auch die Durchmischungsstrecke lang ist.

In Sonderfällen kann durch den Einsatz des Dosierringes auf einen Mischer oder eine Pumpe verzichtet werden.

Beispielsweise kann durch die Zuführung eines Gases, gegebenenfalls eines inerten Gases, Turbulenz in dem Medium erzeugt werden, das das Rohr durchfließt. Somit kann ein sedimentieren einer Suspension durch laminare Strömung im Rohr verhindert werden.

Eine breitere und homogene Verteilung über die gesamte Querschnitt eines Rohrs kann realisiert werden, indem die Dosierstellen bzw. Röhrchen des Dosierringes unterschiedlich lang sind. Damit kann das Edukt gezielt bis in das Rohrinnere dosiert werden.

Die erfindungsgemäße Dosiervorrichtung hat ein breites Anwendungsspektrum. Überall wo schnelle und/oder gleichmäßige Zudosierungen von fließfähigen Medien oder Gasen erforderlich ist. Es können Flüssigkeiten mit niedriger oder hoher Viskosität zudosiert werden, aber auch Suspensionen, Emulsionen, Gase usw. Der Einsatz erfolgt in chemischen Anlagen wie Rohrleitungen oder Rohrreaktoren. Dabei dient der Dosierring als Dosiervorrichtung und/oder Mischer.

In einer besonders bevorzugten Anwendung wird der Dosierring bei der Hydrolyse von Acetoncyanhydrin mit Schwefelsäure zu Methacrylamid verwendet.

Die Erfindung wird durch die folgenden Zeichnungen näher erläutert:
Figur 1
   Längsschnitt durch einen Rohrleitungsabschnitt mit einmontiertem Dosierring
Figur 2
   Vergrößerte Teildarstellung einer abgewandelten Ausführungsform

### Bezugszeichenliste

| | |
|---|---|
| 1 | Rohrleitung |
| 2 | Rohrflansche |
| 3 | Dichtungen |
| 4 | Dosierring |
| 5 | Spannmittel |
| 6 | Verteilerkammer von 4 |
| 7 | Zulaufstutzen für Fluid F |
| 8 | Zulaufstutzen für Fluid F |
| 9 | Injizierung |
| 10 | Innere Wand von Dosierring 4 |
| 11 | Injektionskanäle (Dosierstellen) |
| 12 | Innenwand von 1 |
| 13 | Röhrchen |
| 14 | Anschlagbund für 13 |
| 15 | Austrittkante an 13 |
| 16 | Dosierpunkt |
| | |
| d | Durchmesser von Injektionskanal 11 |
| D | Außendurchmesser von Röhrchen 13 |
| α | Winkel |
| Y | radialer Überstand von 13, 15 |
| M | Medium |
| F | Fluid |

Die Zeichnungen zeigen zwei Ausführungsbeispiele des erfindungsgemäßen Dosierringes.

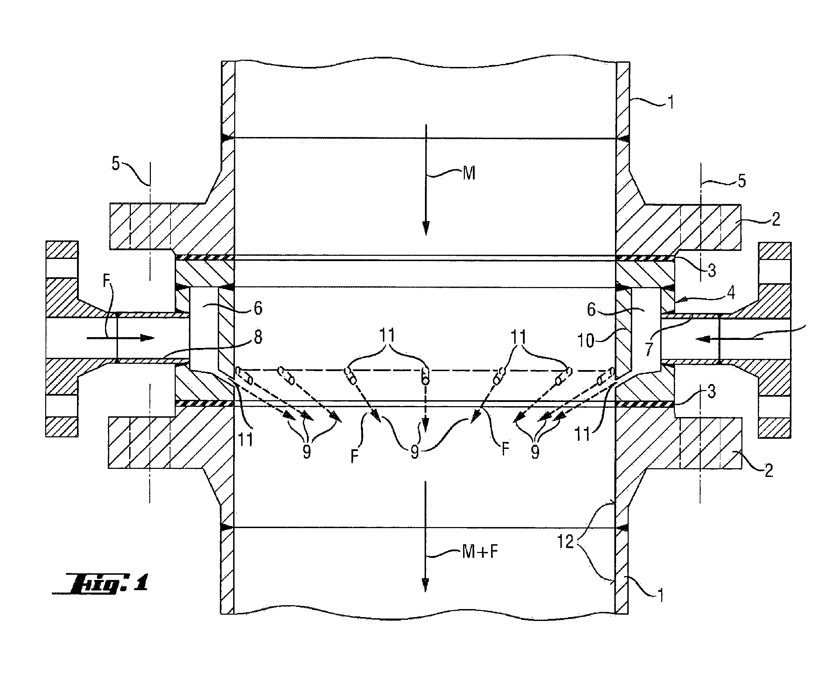

Nach **Fig. 1** ist in eine von einem Medium **M** durchgeströmten Rohrleitung **1** zwischen zwei Rohrflanschen **2** und zwei Dichtungen **3** der erfindungsgemäße Dosierring **4** über schematisch angedeutete Spannmittel 5 einmontiert.

Der Dosierring **4** weist eine umlaufende Verteilerkammer **6** auf, welche von zwei Zulaufstutzen **7** und **8** mit dem zu dosierenden Fluid **F** beströmt wird.

Die innere Wand **10** des Dosierringes **4** wird von vorzugsweise sechzehn gleichmäßig auf den Umfang verteilten Injektionskanälen **11** durchsetzt. Diese sind, wiederum vorzugsweise, unter einem Winkel **α** von 60° gegenüber der Innenwand **12** der **Rohrleitung 1** geneigt.

Hierdurch wird eine gleichmäßige Injizierung **9** des Fluids **F** in den Fluß des Mediums **M** sichergestellt.

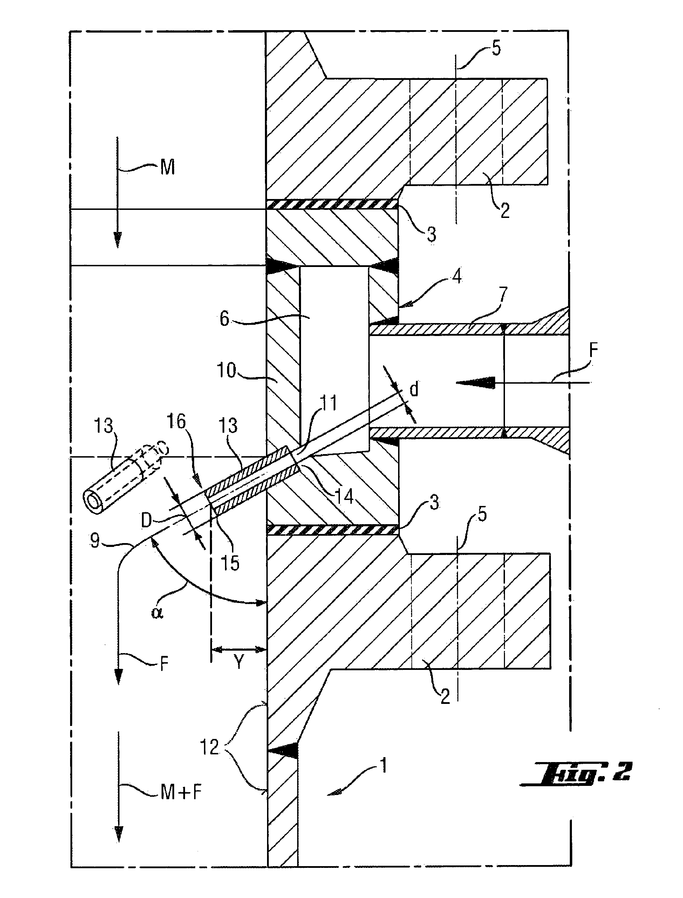

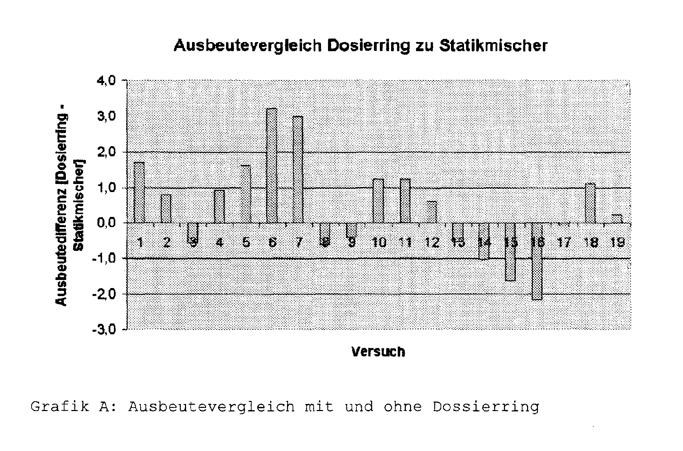

Die Lösung nach **Fig. 2** sieht vor, in die Injektionskanäle **11** Röhrchen **13** einzusetzen, welche einen radialen Überstand **Y** gegenüber der Innenwand **12** der Rohrleitung **1** aufweisen.

Dabei bildet das Ende des Röhrchens **13,** welches damit etwas in den Fluß des Mediums **M** hineinreicht, den Dosierpunkt **16.**

Dadurch wird die Einbringung des Fluids **F** in den Fluß Mediums **M** optimiert, dergestalt, dass das Fluid **F** nicht an der Innenwand **12** der Rohrleitung **1** entlang laufen kann und es am Dosierpunkt **16,** aufgrund der vom Medium **M** umströmten Austrittskanten **15** des Röhrchens **13,** erst im Medienfluß als solchem zum Tropfenabriss kommt.

Damit die Röhrchen **13** nicht bis in die Verteilerkammer **6** eingepresst werden können, ist ein Anschlagbund **14** vorgesehen, welcher durch die Durchmesserdifferenz zwischen dem Durchmesser **d** des Injektionskanals **11** und dem Außendurchmesser **D** des Röhrchens **13** gebildet ist.

Durch dieses Konstruktionsmerkmal ist eine rationelle Montage der Dosierröhrchen **13** gegeben, unter gleichzeitiger Sicherstellung der vorbestimmten Lage des radialen Abstandes **Y** des Dosierpunktes **16** zur Innenwand **12** der Rohrleitung **1.**

Die im Folgenden gegebenen Beispiele werden zur besseren Veranschaulichung der vorliegenden Erfindung gegeben, sind jedoch nicht dazu geeignet, die Erfindung auf die hierin offenbarten Merkmale zu beschränken.

### Beispiele

In mehreren Betriebsversuchen wurden Ausbeutebestimmungen durchgeführt.. Dabei wurde die Wirkung des Dosierrings experimentell bestimmt.

Die Ausbeutebestimmung erfolgte bei einer Verfahrensführung mit Dosierring und einer nachgeschalteten Pumpe als dynamischen Mischer im Vergleich zum herkömmlich eingesetzten statischen Mischer. Die Pumpe ist in Strömungsrichtung unmittelbar nach dem Dosierring angeordnet (nachgeschaltet). Der Dosiering ist direkt an den Ansaugstutzen der Pumpe geflanscht (montiert) um einen möglichst kurzen Weg des ACH's bis zur Vermischung zu gewährleisten und somit eine möglichst schnelle Vermischung zu erreichen. Die Pumpe, bei der Verfahrensführung mit dem erfindungsgemäßen Dosierring, ist eine Umwälzpumpe, die im Schlaufenreaktor üblicherweise zur Kreislaufführung des Amidgemisches genutzt wird.

Die erhaltenen Ausbeuten werden in der Grafik A gegenübergestellt. Es wurden verschiedene Versuchsparameter variiert und die Ausbeuten durch Probennahmen und Analysen des Amidgemisches bestimmt.

Die Mehrzahl der Messungen ergeben eine Ausbeuteerhöhung. Positive Ausbeutedifferenzen bis zu 3,2 % zeigen eine Verbesserung der Ausbeuten durch den Einsatz des Dosierrings.

## Patentansprüche

1. Vorrichtung zur Dosierung von fließfähigen Medien oder Gasen, **dadurch gekennzeichnet, dass** ein oder mehrere Dosierringe mit Dosierstellen [11] versehen in Rohrleitungen, Rohrreaktoren oder Schlaufenreaktoren eingesetzt werden
wobei der oder die Dosierringe außenliegend sind, eine umlaufende Verteilerkammer [6] aufweisen und die innere Wand der Dosierringe von 2 bis 20 Injektionskanälen durchsetzt ist.

2. Vorrichtung nach Anspruch 1, **dadurch gekennzeichnet, dass** die Dosierstellen in verschiedensten Orientierungen und Längen realisiert werden können

3. Vorrichtung nach Anspruch 1, **dadurch gekennzeichnet, dass** die fließfähigen Medien oder Gase mit Überdruck über die Dosierstellen zugegeben werden.

4. Vorrichtung nach Anspruch 1, **dadurch gekennzeichnet, dass** der Dosierring temperiert wird.

5. Verwendung der Dosiervorrichtung nach Anspruch 1 in chemischen Verfahren mit schnellen Mischvorgängen.

6. Verwendung der Dosiervorrichtung nach Anspruch 4 zur Herstellung von Methacrylamid aus Acetoncyanhydrin und Schwefelsäure.

7. Verwendung der Dosiervorrichtung nach Anspruch 1 in Öl- und Gaspipelines sowie Trink-, Brauch- oder Abwasserleitungen.

8. Verwendung der Dosiervorrichtung nach Anspruch 1 in Rohrleitungen an Einspeisungsstellen fließfähiger oder gasförmiger Medien.

## Claims

1. Apparatus for metering free-flowing media or gases, **characterized in that** one or more metering rings with metering sites [11] provided in pipelines, tubular reactors or loop reactors are used,
wherein the metering ring is or metering rings are on the outside and has or have a peripheral distribution chamber [6], and the inner wall of the metering rings is penetrated by 2 to 20 injection channels.

2. Apparatus according to Claim 1, **characterized in that** the metering sites can be implemented in a wide variety of different orientations and lengths.

3. Apparatus according to Claim 1, **characterized in that** the free-flowing media or gases are added via the metering sites at elevated pressure.

4. Apparatus according to Claim 1, **characterized in that** the temperature of the metering ring is controlled.

5. Use of the metering apparatus according to Claim 1 in chemical processes with rapid mixing operations.

6. Use of the metering apparatus according to Claim 4 for preparing methacrylamide from acetone cyanohydrin and sulphuric acid.

7. Use of the metering apparatus according to Claim 1 in oil and gas pipelines, and in drinking water, service water or wastewater conduits.

8. Use of the metering apparatus according to Claim 1 in pipelines at feed sites for free-flowing or gaseous media.

## Revendications

1. Dispositif pour le dosage de milieux liquides ou de gaz, **caractérisé en ce qu'**une ou plusieurs bague(s) de dosage dotées de points de dosage (11) est/sont utilisée(s) dans des conduites tubulaires, des réacteurs tubulaires ou des colonnes à écoulement en boucle, dans lequel la ou les bague(s) de dosage est/sont située(s) à l'extérieur, présente(nt) une chambre de répartition périphérique (6) et la paroi intérieure de la/des bague(s) de dosage est traversée par 2 à 20 canaux d'injection.

2. Dispositif selon la revendication 1, **caractérisé en ce que** les points de dosage peuvent être réalisés avec des orientations et des longueurs très différentes.

3. Dispositif selon la revendication 1, **caractérisé en ce que** les milieux coulants ou les gaz sont fournis avec une surpression via les points de dosage.

4. Dispositif selon la revendication 1, **caractérisé en ce que** la bague de dosage est tempérée.

5. Utilisation du dispositif de dosage selon la revendication 1 dans des procédés chimiques avec des opérations de mélange rapides.

6. Utilisation du dispositif de dosage selon la revendication 4 pour la production de méthacrylamide à partir de cyanohydrine d'acétone et d'acide sulfurique.

7. Utilisation du dispositif de dosage selon la revendication 1 dans des oléoducs et des gazoducs ainsi que dans des canalisations d'eau potable, d'eau sanitaire ou d'eaux usées.

8. Utilisation du dispositif de dosage selon la revendication 1 dans des conduites tubulaires à des points d'alimentation de milieux liquides ou gazeux.
